# EUROPEAN PATENT SPECIFICATION

(11) **EP 2 301 615 B1**
(45) Date of publication and mention of the grant of the patent: **22.02.2012**
(21) Application number: 09425382.0
(22) Date of filing: 29.09.2009
(51) Int. Cl.: A61M 16/10

(54) **Respiratory device for medication delivery**
Atemgerät zur Abgabe von Arzneimitteln
Dispositif respiratoire pour administration de médicaments

(43) Date of publication of application: 30.03.2011
(73) Proprietor: Covidien AG, 8212 Neuhausen am Rheinfall (CH)
(72) Inventor: Zucchi, Giuseppe, 41039 San Possidonio (Modena) (IT); Resca, Daniele, 41038 San Felice Sul Panaro (Modena) (IT); Romagnoli, Elisa, 44042 Cento (Ferrara) (IT)
(74) Representative: Firmati, Leonardo

(56) References cited:
- US-A- 5 848 587
- US-A1- 2004 123 974
- US-A1- 2006 157 056
- US-A1- 2006 219 243
- US-B1- 7 069 928

## Description

The present invention relates to a respiratory device for medication delivery.

In particular the present invention relates to a respiratory device capable of exchanging heat and moisture and/or filtering respiratory gases in combination with an aerosol additional path.

More in particular the present invention is suitable for use connected to equipment for mechanical ventilation.

During mechanical ventilation, the air flow supplied by the ventilator to the patient shall have adequate temperature level and moisture content because the patient has bypassed upper airways due to tracheal intubation.

Heat and humidity can be added to respiratory gases through active humidifiers. In alternative, prevention of heat and moisture loss from airways can be achieved by means of devices called HME (Heat and Moisture Exchanger) or artificial noses able to trap heat and humidity during exhalation and returning good portion of the same to the patient in inhalation phase. In addition respiratory gases can be filtered through suitable means called antibacterial/antiviral filters. These respiratory devices are therefore fitted with suitable elements able to act as heat and moisture exchangers and filters.

In the following description and claims, the terms "air" and "respiratory gases" will be both referred to as ventilation gases which may be indifferently normal air or another gas mixture.

Frequently, a patient connected to a mechanical ventilator also requires aerosol therapy.

It is important that the flow of air conveying the nebulized medication does not pass through the heat and moisture exchanger (HME) and/or filter because the medication would otherwise be partially absorbed by the HME. This would result in a waste of medication, inefficiency of treatment and also lead to deterioration and clogging of the exchanger/filtering element.

The respiratory devices currently available on the market comprises an HME element positioned along the main channel and an alternative path excluding the heat and moisture exchanger bypassing it.

Inside, these devices have a selector that directs the air flow only, when the humidification mode is required, and switcher the air flow towards the Bypass when the alternative medication mode is selected.

For example, in documents US 2006/219243 A1, US 2004/123974 A1, US 7 069 928 B1, US 2006/157056 A1 and US 5 848 587 A are shown respiratory devices which present heat-moisture exchangers with aerosol bypass, but that are clearly not easy to use Indeed, they present different parts that have to be contemporary handled, kept still and turned by an operator.

A drawback pointed out is that the diverter must be switched manually by the operator Selecting from one operating position to another is not always easy especially if the device is being operated by one person alone which is often the case in hospitals or when patients are receiving treatment at home.

During operation of the diverter, the device must be kept in position by the operator ensuring that it does not detach from the two portion of respiratory circuit that conveys air from the ventilator to the patient

The devices which are currently commercially available have at least three components to be handled during the switching phase and this factor clearly complicates matters for a person operating the device alone.

The technical purpose of the present invention is to overcome the drawbacks described above by providing a respiratory device capable of exchanging heat and moisture and/or filtering

respiratory gases coupled with an alternative tool for medication delivery.

In particular, the present invention is aimed to provide a respiratory device capable of exchanging heat and moisture and/or filtering respiratory gases in combination with an additional path for aerosol delivery which is simple and inexpensive to manufacture.

Another aim of the invention is to provide a respiratory device capable of exchanging heat and moisture and/or filtering respiratory gases in combination with an additional path for aerosol delivery which is easy for one person to operate alone and which provides a clear visual indication of the current operating position of the flow diverter.

The technical purpose and aims specified are substantially achieved by a respiratory device capable of exchanging heat and moisture and/or filtering respiratory gases in combination with an additional path for aerosol delivery, comprising the technical features described in one or more of the claims below.

Further features and advantages of the present invention will become apparent from the description below of a preferred embodiment shown in the accompanying drawings, given purely by way of a non-limiting example, in which:
- Figure 1 is a perspective view of the device according to the present invention;
- Figure 2 is an exploded view of the device shown in Figure 1;
- Figure 3 is a side cross-section of the device in a first operating position;
- Figure 4 is a side cross-section of the device in a second operating position.

In the drawings, the numeral 1 denotes in its entirety a respiratory device capable of exchanging heat and moisture and/or filtering respiratory gases in combination with an additional path dedicated to convey aerosol.

The respiratory device 1 is positioned along a breathing circuit between Y-piece and patient interface (endotracheal or tracheostomy tube) conveying ventilation gases to a patient.

The device 1 is connected to the respiratory circuit by a tubular fitting 5 which is open at its opposite ends 5b and 5c to allow the passage of fluid.

In this way the respiratory circuit is divided into a first section upstream, located between the ventilator and the device, and a second section downstream the device, located between the device and the patient.

The device 1 comprises a first chamber 2 having two openings 2b', 2b", one for the inlet and one for the outlet of air flow.

Both of the openings 2b', 2b" act as inlet and outlet sections depending on whether the patient is inhaling or exhaling.

The first chamber 2 contains an inner member 30 through which the respiratory gases flow from ventilator to the patient and vice-versa.

In the preferred embodiment shown in the figures the inner member 30 comprises a heat and moisture exchanger element 3 and a filter element 9. According to different embodiments (not shown) of the present invention, the inner member 30 comprises either a heat and moisture exchanger element 3 or a filter element 9.

The flow passes through the heat and moisture exchanger element 3 and the filter element 9 and therefore the first chamber 2 during exhalation and inhalation.

The exchanger element 3 is a cylinder made of an advantageously porous material designed to retain the heat and moisture contained in the exhaled air and return good portion of the same to inhaled gases, usually cold and dry.

In a preferred embodiment, the inside of the first chamber 2, housing the heat and moisture exchanger element 3, can alternatively or additionally have an antibacterial/antiviral filter element 9 acting to guarantee the good quality of the respiratory gases inhaled by the patient.

The device 1 also comprises a second chamber 4 defining an alternative channel for a flow of air mixed with a nebulized medication.

Advantageously, the chamber 4 can be used when the patient is undergoing aerosol therapy.

The second chamber 4 also has two openings 4b', 4b" for the passage of the air/medication flow. Similarly to the first chamber 2, the second chamber 4 has two openings 4b', 4b" acting as the inlet or outlet section for the air/medication flow depending on whether the patient is inhaling or exhaling.

During aerosol therapy, the medication in suspension is directed into the air flow. It is preferable that the flow of air and medication does not pass through the filter element or the exchanger element because of the possibility of retainment at least of some medication.

The tubular fitting 5 by which the device 1 is connected to the respiratory circuit is positioned between the first chamber 2 and the second chamber 4.

In addition, the tubular fitting 5 has at least two side openings 6, one being an inlet and the other an outlet, for the fluid connection with the first chamber 2 or with the second chamber 4.

Advantageously, the tubular fitting 5 is placed in fluid communication alternately with the first chamber 2 or the second chamber 4 by aligning the side openings 6 of the tubular fitting 5 with the openings 2b', 2b" of the first chamber 2 or with the openings 4b', 4b" of the second chamber 4 according to whether normal respiration or aerosol therapy is in progress.

In the present description and claims, the expression "in fluid communication" referred to different parts means that there is a fluid connection between said parts.

When the device 1 is operative, the tubular fitting 5 is in stable, fluid communication with the respiratory circuit.

The two chambers 2 and 4 are therefore placed alternately in fluid communication with the respiratory circuit to which the device 1 is connected.

The inside of the tubular fitting 5 comprises a fluid diverter 7 for diverting the flow of air or air/medication from the axial direction to the radial direction towards the outside of the tubular fitting 5. Depending on the position of the device 1, the flow diverter 7 diverts the flow of air towards the first chamber 2 or the flow of air/medication towards the second chamber 4 by placing the first chamber 2 and the second chamber 4 selectively and alternately in fluid communication with the respiratory circuit to which the device 1 is connected.

The flow diverter 7 is an element positioned inside the tubular fitting 5 which interrupts the flow inside the tubular fitting 5 along the axis 5a of the fitting and causes the flow to exit through one of the side openings 6.

This means that the flow of air that enters the tubular fitting 5 leaves from the first of the two side openings 6 that it encounters, flows through the first chamber 2 or second chamber 4, re-enters the fitting through the other side opening 6, runs along the last section of the tubular fitting 5 then flow along the respiratory circuit towards the patient during the inhalation phase or towards the ventilator during the exhalation phase.

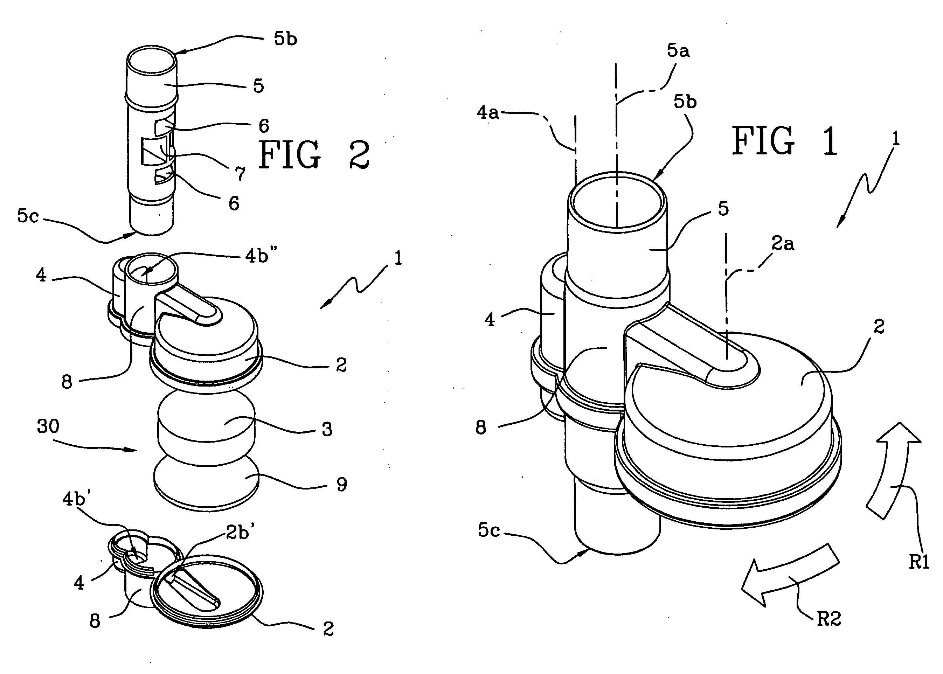

The first chamber 2 and the second chamber 4 are structurally connected to each other by a central coupling sleeve 8 to form a single block as shown in Figures 1 and 2.

The coupling sleeve 8 is fitted over the tubular fitting 5 and can be turned around it between a first operating position in which the air flows through the first chamber 2 and a second operating position in which the air/medication flows through the second chamber 4.

Therefore, both the chambers 2 and 4 at the sides of the tubular fitting 5 can rotate around the axis 5a while maintaining their respective axes 2a, 4a and 5a parallel to each other.

In use, the device is located on the respiratory circuit and is connected to it by the ends 5b and 5c of the tubular fitting 5.

A visual indicator such as a mark on the outside of the device indicates to the operator the current operating mode of the device.

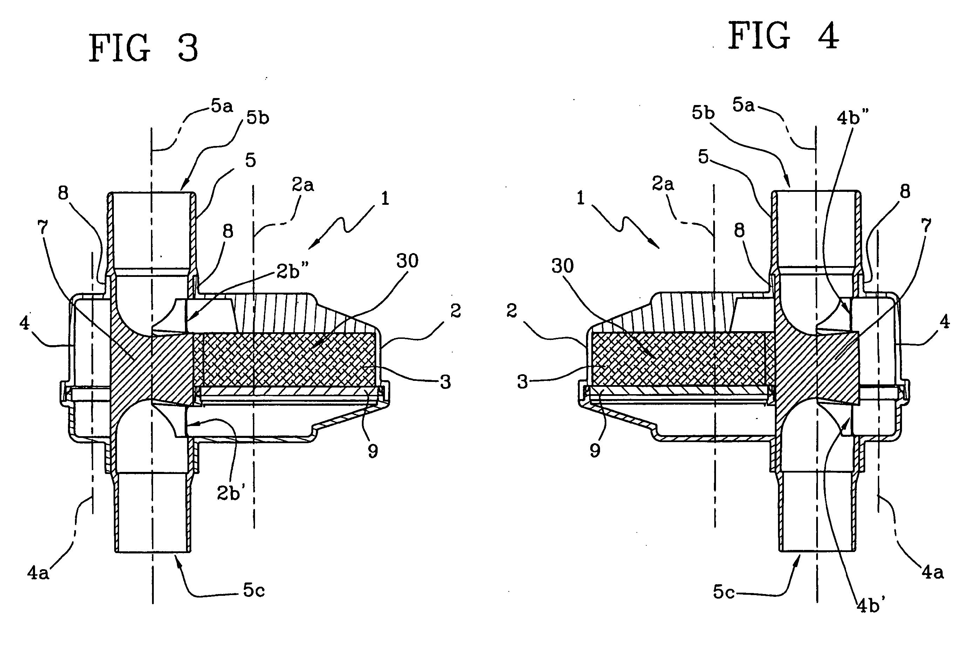

In the first operating position, corresponding to normal respiratory phase shown in Figure 3, the tubular fitting 5 is in fluid communication with the first chamber 2. This means that the side openings 6 of the tubular fitting 5 are aligned with the openings 2b', 2b" of the first chamber 2. The openings 4b' , 4b" of the second chamber 4 are closed by the external side wall of the tubular fitting 5. In this way the second chamber 4 is completely isolated.

During the inhalation phase, the flow of air coming from the ventilator enters the tubular fitting 5, flows into the first chamber 2, passes through the inner member 30 (i.e. the heat and moisture exchanger element 3 and the bacterial filter element 9), leaves the chamber and passes through the final section of the tubular fitting 5 and then runs along the respiratory circuit towards the patient.

During the exhalation phase the path followed is the same but the air flow is in the opposite direction.

To use the second operating mode, that is, to set the device for the aerosol therapy mode, the single block formed by the two chambers 2, 4 and the sleeve 8 is rotated through 180° around the axis 5a of the tubular fitting 5 in the direction R1 or R2 as shown in Figure 1.

In this way the openings 4b', 4b" of the second chamber 4 are aligned with the side openings 6 of the tubular fitting 5.

The first chamber 2 is in turn closed by the outer wall of the tubular fitting 5.

In other words, in the second operating position, corresponding to aerosol therapy phase shown in Figure 4, the tubular fitting 5 is in fluid communication with the second chamber 4.

During inhalation, the flow of air/medication runs along a first section of the tubular fitting 5, through the second chamber 4, along the second section of the tubular fitting 5 and then finally through the respiratory circuit and onwards to the patient. During exhalation the path travelled is the same but the flow is in the opposite direction. As clearly described above, the second chamber 4 defines an additional path for medication delivery. The present invention has notable advantages and achieves the purposes described above.

A device according to the present invention is very simple and inexpensive to manufacture.

In addition, it is easy for a single operator to use and can be switched from one operating position to another without difficulty.

This is because there are only two main components to be rotated. The operator simply holds the tubular fitting with one hand and rotates the single block comprising the two chambers with the other. This is done without difficulty and there is no risk of moving the device from its correct operating position.

## Claims

1. A respiratory device, connected to a respiratory circuit, having an additional path for medication delivery, comprising a first chamber (2) containing a inner member (30) and having two openings (2b', 2b") for the inlet and outlet of air flow, a second chamber (4) defining the additional path and having two other openings (4b', 4b") for the inlet and outlet of air/medication flow, a tubular fitting (5) being able to place in fluid communication with either the first chamber (2) or the second chamber (4) and having an internal flow diverter (7) for deflecting the air flow towards the first chamber (2), via openings (2b', 2b"), when said first chamber (2) is in fluid communication with the respiratory circuit and towards the second chamber (4), via the two other openings (4b', 4b"), when said second chamber (4) is in fluid communication with the respiratory circuit; **characterized in that** said tubular fitting (5) is positioned between the first chamber (2) and the second chamber (4), open at its opposite ends (5b, 5c) to connect the device (1) to the respiratory circuit; said first chamber (2) and said second chamber (4) being able to turn around the tubular fitting (5) between a first operating position in which the air flows through the first chamber (2) and a second operating position in which the air/medication flows through the second chamber (4) .

2. The device according to any of the foregoing claims, **characterized in that** the tubular fitting (5) comprises at least two side openings (6), one being an inlet and the other an outlet, for the fluid connection with the inlet and outlet openings (2b', 2b") of the first chamber (2) or with the inlet and outlet openings (4b', 4b") of the second chamber (4).

3. The device according to any of the foregoing claims, **characterized in that** the first chamber (2) and the second chamber (4) are structurally connected to each other by a central coupling sleeve (8).

4. The device according to claim 3, **characterized in that** the coupling sleeve (8) is fitted over the tubular fitting (5) and can be turned around it between a first operating position in which the air flows through the first chamber (2) and a second operating position in which the air/medication flows through the second chamber (4).

5. The device according to any of claims 1 to 4, **characterized in that** said inner member (30) comprises an HME element (3).

6. The device according to any of claims 1 to 4, **characterized in that** said inner member (30) comprises a bacterial filter element (9).

7. The device according to any of claims 1 to 4, **characterized in that** said inner member (30) comprises a combination of an HME element (3) and a bacterial filter element (9).

8. A ventilation system **characterised in that** it comprises a respiratory device as set out in any of claims 1 to 7.

## Patentansprüche

1. Atemgerät, das mit einem Atemkreislauf verbunden ist, aufweisend eine zusätzliche Bahn für die Abgabe von Arzneimitteln, umfassend eine erste Kammer (2), die ein inneres Glied (30) umfasst und zwei Öffnungen (2b', 2b") für den Einlass und Auslass einer Luftströmung enthält, eine zweite Kammer (4), welche die zusätzliche Bahn definiert und zwei weitere Öffnungen (4b', 4b") für den Einlass und Auslass einer Luft/Arzneimittelströmung aufweist, ein rohrförmiges Anschlussstück (5), das dazu fähig ist, sich mit entweder der ersten Kammer (2) oder der zweiten Kammer (4) in Fluidverbindung zu setzen und aufweisend einen internen Strömungsableiter (7) zum Umlenken der Luftströmung zur ersten Kammer (2) durch Öffnungen (2b', 2b"), wenn die erste Kammer (2) in Fluidverbindung mit dem Atemkreislauf steht, und zur zweiten Kammer (4) durch die beiden weiteren Öffnungen (4b', 4b"), wenn die zweite Kammer (4) in Fluidverbindung mit dem Atemkreislauf steht; **dadurch gekennzeichnet, dass** das rohrförmige Anschlussstück (5) zwischen der ersten Kammer (2) und der zweiten Kammer (4) positioniert ist und an seinen gegenüberliegenden Enden (5b, 5c) offen ist, um das Gerät (1) mit dem Atemkreislauf zu verbinden; wobei die erste Kammer (2) und die zweite Kammer (4) dazu fähig sind, sich um das rohrförmige Anschlussstück (5) zwischen einer ersten Betriebsposition, in der die Luft durch die erste Kammer (2) strömt, und einer zweiten Betriebsposition, in der die Luft/das Arzneimittel durch die zweite Kammer (4) strömt, zu drehen.

2. Gerät nach einem der vorangehenden Ansprüche, **dadurch gekennzeichnet, dass** das rohrförmige Anschlussstück (5) mindestens zwei seitliche Öffnungen (6), von denen eine ein Einlass und die andere ein Auslass ist, für die Fluidverbindung mit den Einlass- und Auslassöffnungen (2b', 2b") der ersten Kammer (2) oder mit den Einlass- und Auslassöffnungen (4b', 4b") der zweiten Kammer (4) umfasst.

3. Gerät nach einem der vorangehenden Ansprüche, **dadurch gekennzeichnet, dass** die erste Kammer (2) und die zweite Kammer (4) strukturell miteinander durch eine zentrale Verkupplungsmuffe (8) verbunden sind.

4. Gerät nach Anspruch 3, **dadurch gekennzeichnet, dass** die Verkupplungsmuffe (8) auf das rohrförmige Anschlussstück (5) aufgesetzt ist und zwischen einer ersten Betriebsposition, in der die Luft durch die erste Kammer (2) strömt, und einer zweiten Betriebsposition, in welcher die Luft/das Arzneimittel durch die zweite Kammer (4) strömt, darum gedreht werden kann.

5. Gerät nach einem der Ansprüche 1 bis 4, **dadurch gekennzeichnet, dass** das innere Glied (30) ein HME-Element (3) umfasst.

6. Gerät nach einem der Ansprüche 1 bis 4, **dadurch gekennzeichnet, dass** das innere Glied (30) ein Bakterienfilterelement (9) umfasst.

7. Gerät nach einem der Ansprüche 1 bis 4, **dadurch gekennzeichnet, dass** das innere Glied (30) eine Kombination eines HME-Elements (3) und eines Bakterienfilterelements (9) umfasst.

8. Belüftungssystem, **dadurch gekennzeichnet, dass** es ein Atemgerät umfasst, das in einem der Ansprüche 1 bis 7 dargelegt wird.

## Revendications

1. Dispositif respiratoire, raccordé à un circuit respiratoire, avec un parcours supplémentaire pour administration de médicaments, comprenant une première chambre (2) avec un organe interne (30) et avec deux ouvertures (2b', 2b") pour l'entrée et pour la sortie de flux d'air, une seconde chambre (4) déterminant le parcours supplémentaire et avec deux autres ouvertures (4b', 4b") pour l'entrée et pour la sortie de flux d'air/de médicament, un raccord tubulaire (5) pouvant se mettre en communication fluidique avec, soit la première chambre (2), soit la seconde chambre (4) et avec un déflecteur de flux interne (7) pour dévier le flux d'air en direction de la première chambre (2), par des ouvertures (2b', 2b"), lorsque ladite première chambre (2) est en communication fluidique avec le circuit respiratoire, et en direction de la seconde chambre (4), par les deux autres ouvertures (4b', 4b"), lorsque ladite seconde chambre (4) est en communication fluidique avec le circuit respiratoire ; **caractérisé en ce que** ledit raccord tubulaire (5) est positionné entre la première chambre (2) et la seconde chambre (4), ouvert à ses extrémités opposées (5b, 5c), pour raccorder le dispositif (1) au circuit respiratoire ; ladite première chambre (2) et ladite seconde chambre (4) pouvant tourner autour du raccord tubulaire (5) entre une première position opérationnelle dans laquelle l'air traverse la première chambre (2) et une seconde position opérationnelle dans laquelle l'air/le médicament traverse la seconde chambre (4).

2. Dispositif selon l'une quelconque des revendications précédentes, **caractérisé en ce que** le raccord tubulaire (5) comprend au moins deux ouvertures latérales (6), l'une avec une entrée et l'autre avec une sortie, pour le raccordement fluidique avec les ouvertures d'entrée et de sortie (2b', 2b") de la première chambre (2) ou avec les ouvertures d'entrée et de sortie (4b', 4b") de la seconde chambre (4).

3. Dispositif selon l'une quelconque des revendications précédentes, **caractérisé en ce que** la première chambre (2) et la seconde chambre (4) sont structurellement raccordées l'une à l'autre par un manchon d'accouplement central (8).

4. Dispositif selon la revendication 3, **caractérisé en ce que** le manchon d'accouplement (8) est monté sur le raccord tubulaire (5) et peut tourner autour de lui entre une première position opérationnelle dans laquelle l'air traverse la première chambre (2), et une seconde position opérationnelle dans laquelle l'air/le médicament traverse la seconde chambre (4).

5. Dispositif selon l'une quelconque des revendications 1 à 4, **caractérisé en ce que** ledit organe interne (30) comprend un élément HME (3).

6. Dispositif selon l'une quelconque des revendications 1 à 4, **caractérisé en ce que** ledit organe interne (30) comprend un élément filtrant antibactérien (9).

7. Dispositif selon l'une quelconque des revendications 1 à 4, **caractérisé en ce que** ledit organe interne (30) comprend une association entre un élément HME (3) et un élément filtrant antibactérien (9).

8. Système de ventilation **caractérisé en ce qu'**il comprend un dispositif respiratoire selon l'une quelconque des revendications 1 à 7.
